# FASCICULE DE BREVET EUROPEEN

(11) **EP 2 912 049 B1**
(45) Date de publication et mention de la délivrance du brevet: **25.05.2016**
(21) Numéro de dépôt: 13779313.9
(22) Date de dépôt: 26.09.2013
(51) Int. Cl.: C07H 1/00, C07H 3/04, C12N 1/38, C12N 9/42

(54) **PROCÉDÉ DE PRODUCTION DE SOPHOROSE À PARTIR DE SOPHOROLIPIDES**
VERFAHREN ZUR HERSTELLUNG VON SOPHOROSE AUS SOPHOROLIPIDEN
METHOD FOR PRODUCING SOPHOROSE FROM SOPHOROLIPIDS

(30) Priorité: 29.10.2012 FR 1202892
(43) Date de publication de la demande: 02.09.2015
(73) Titulaire: IFP Énergies nouvelles, 92852 Rueil-Malmaison Cedex (FR)
(72) Inventeur: JOURDIER, Etienne, F-92420 Vaucresson (FR); BEN CHAABANE, Fadhel, F-75020 Paris (FR)
(86) Numéro de dépôt international: PCT/FR2013/052269
(87) Numéro de publication internationale: WO 2014/068208

(56) Documents cités:
- ES-A1- 2 103 688

## Description

La présente invention concerne un procédé de production de sophorose par hydrolyse acide de sophorolipides. L'invention a également pour objet l'utilisation d'un hydrolysat obtenu selon le procédé de l'invention comme solution inductrice de l'expression de gènes codant pour des cellulases. Enfin l'invention se rapporte à un procédé de purification du sophorose à partir de l'hydrolysat.

### Etat de la technique

Les cellulases adaptées à l'hydrolyse des polysaccharides permettent aux microorganismes qui les produisent d'utiliser la cellulose et l'hémicellulose (composants majoritaires des végétaux) comme source de carbone, en hydrolysant ces polymères en sucres simples (glucose, xylose, etc).

A l'heure actuelle, ces enzymes sont mises en oeuvre dans de nombreux domaines tels que dans la détergence, l'industrie textile ou alimentaire, ainsi que dans les procédés visant la valorisation biochimique de la biomasse lignocellulosique (par exemple pour la production d'éthanol biocarburant de seconde génération). *Trichoderma reesei* est aujourd'hui reconnu comme le meilleur microorganisme producteur de cellulases dans des procédés de production de biocarburant à partir de biomasse lignocellulosique en raison principalement de son fort pouvoir de sécrétion.

Cependant le coût de production des cellulases reste élevé et représente un des obstacles économiques pour une mise en oeuvre à l'échelle industrielle des procédés dits de seconde génération.

Industriellement, la production optimisée de cellulases par *Trichoderma reesei* est réalisée en protocole fed-batch (alimentation sans soutirage) en utilisant une solution d'alimentation contenant du lactose comme sucre inducteur de la production de cellulases (EP 448 430 B1). Bien qu'utilisé industriellement, le lactose présente l'inconvénient d'être coûteux pour permettre une production de cellulases à bas prix.

Le sophorose est connu pour être un très bon inducteur du système cellulolytique de *Trichoderma reesei.* En 1962, Mandels M. et al. [Sophorose as an inducer of cellulase in Trichoderma viride. Journal of Bacteriology 1962, 83:400-408] ont démontré que le sophorose, alors qu'il était présent seulement à hauteur de 0.006% comme impureté dans du glucose, permettait l'induction de la production d'enzymes cellulolytiques (ou cellulases). Ces impuretés de sophorose étaient formées par un mécanisme de transglycosylation lors de l'hydrolyse acide de l'amidon en glucose.

Le sophorose (2-O-β-D-Glucopyranosyl-D-glucose) est un diholoside constitué de deux unités glucose reliées par une liaison osidique β(1→2). Il est présent dans la nature, par exemple dans la gousse de l'arbre *Sophora japonica,* à partir de laquelle il a été isolé pour la première fois en 1938.

Malheureusement, le prix élevé du sophorose fait qu'il n'est pas encore possible de l'utiliser à l'échelle industrielle. A ce jour il est uniquement mis en oeuvre en laboratoire pour des expériences nécessitant une forte induction.

Un but de la présente invention est de remédier aux inconvénients décrit ci-dessus et en particulier de proposer un procédé simple et économique de production de sophorose à partir de sophorolipides. Le sophorose contenu dans l'hydrolysat peut alors être utilisé comme agent inducteur chez des microorganismes capables de produire des cellulases, et en particulier chez *Trichoderma reesei.*

### Résumé de l'invention

Ce but est atteint grâce à un procédé dans lequel :
a) on fournit une solution aqueuse de sophorolipides;
b) on ajoute un composé acide dans la solution aqueuse de sophorolipides, la teneur en composé acide étant comprise entre 0,01% et 6% poids par rapport au poids de sophorolipides;
c) on chauffe le mélange obtenu à l'étape b) pendant 2 à 200 heures et à une température comprise entre 60 et 110°C.

Le procédé selon l'invention met en oeuvre une hydrolyse de sophorolipide en milieu aqueux en présence d'un composé acide, c'est-à-dire qui est capable de libérer des ions H⁺ en solution.

Dans le cadre de la présente invention, le terme sophorolipide (ou sophorose lipide) désigne des composés comprenant une unité sophorose reliée à un acide gras par une liaison de type osidique (entre le carbone 1' du sophorose et celui en ω-1 de l'acide gras). Les sophorolipides peuvent être produits par des microorganismes, en particulier par des levures du genre *Candida.* Selon le lipide fourni au microorganisme et les conditions de culture, on peut obtenir un mélange de différents sophorolipides, sous forme acide (fonction carboxylique libre) ou lactone cyclique (liaison ester entre l'acide gras et le groupement -OH en 4" du sophorose), et avec les groupements -OH 6' et 6" qui peuvent être acétylés.

La teneur en sophorolipides du milieu est généralement comprise entre 2 et 800 g/L, et de préférence entre 100 et 400 g/L.

La teneur en composé acide est comprise entre 0,01% et 6% poids par rapport au poids de sophorolipides, de préférence, comprise entre 0,1 et 2% poids par rapport au poids de sophorolipides. De manière encore plus préférée, la teneur en composé acide est comprise entre 0,1 et 1% poids par rapport au poids de sophorolipides.

Selon un mode de réalisation particulier, la teneur en sophorolipide du milieu est comprise entre 100 et 400 g/L et la teneur en composé acide est comprise entre 0,1 et 2% poids par rapport au poids de sophorolipides.

Le composé acide mis en oeuvre est un acide inorganique ou un acide organique.

De préférence le composé acide est un acide inorganique choisi parmi l'acide sulfurique, l'acide chlorhydrique, l'acide phosphorique, l'acide nitrique. De préférence, l'acide inorganique est de l'acide sulfurique.

Selon un mode de réalisation particulier du procédé, on effectue en outre une étape de séparation de la phase lipide et de la phase aqueuse du mélange issu de l'étape d'hydrolyse de manière à récupérer une solution aqueuse (hydrolysat) contenant du sophorose et du glucose.

L'hydrolysat ainsi obtenu à l'issue de l'étape d'hydrolyse et après séparation de la phase lipide est utilisé comme solution pour induire l'expression de gènes codant pour des cellulases chez un microorganisme tel que par exemple *Trichoderma reesei.*

Avant sa mise en oeuvre comme solution inductrice, on peut également purifier ladite solution aqueuse contenant le mélange de sophorose et de glucose en ajoutant à ladite solution un microorganisme alcooligène capable de fermenter le glucose en éthanol puis en effectuant une fermentation éthanolique et enfin en réalisant une séparation de l'éthanol (par exemple par stripage) afin de récupérer un hydrolysat purifié contenant du sophorose.

### Description détaillée de l'invention

La présente invention décrit les conditions de mise en oeuvre d'une hydrolyse acide ménagée dans le but de produire du sophorose et en évitant l'hydrolyse totale des sophorolipides qui conduirait à la libération de deux unités glucose par molécule de sophorolipide.

Dans les conditions décrites ci-dessous, l'hydrolyse permet de rompre préférentiellement la liaison osidique entre le sophorose et la chaine lipidique, ce qui aboutit à la libération de sophorose. Cependant, inévitablement et dans une plus ou moins forte mesure en fonction des conditions opératoires, le sophorose libéré peut lui aussi être hydrolysé en deux unités glucose.

En pratique, on fournit d'abord une solution aqueuse de sophorolipides. Cette solution aqueuse présente typiquement une concentration en sophorolipides comprise entre 2 et 800 g/L, de préférence entre 100 et 400 g/L. De manière préférentielle, on utilisera des sophorolipides sous forme acide plutôt que sous forme lactone. La forme acide peut être obtenue soit en optimisant les conditions de fermentation lors de la production des sophorolipides, soit en réalisant une hydrolyse en conditions basiques (saponification) du produit de la fermentation.

A cette solution aqueuse on ajoute un composé acide de manière à avoir une concentration en acide dans le mélange comprise entre 0,01% et 6% poids par rapport au poids de sophorolipides, et de préférence en 0,1 et 2% poids et de manière plus préférée entre 0,1 et 1% poids.

Le composé acide est soit un acide organique soit un acide inorganique. De préférence on emploie un acide inorganique qui peut être choisi parmi l'acide sulfurique, l'acide chlorhydrique, l'acide phosphorique, l'acide nitrique. De préférence, l'acide est l'acide sulfurique. Si un acide organique est utilisé, il peut être choisi parmi l'acide formique, l'acide acétique, l'acide oxalique, l'acide trifluoroacétique et l'acide maléique.

L'hydrolyse est réalisée en portant le mélange acide à une température généralement comprise entre 60°C et 110°C pendant 2 heures à 200 heures, de préférence entre 5 heures et 24 heures. A l'issue de ce traitement les acides gras libérés par l'hydrolyse forment une phase non miscible avec l'eau qui peut être facilement séparée de la phase aqueuse. La phase aqueuse (appelée hydrolysat) contient un mélange de glucose et de sophorose dont les proportions varient en fonction de la dose d'acide utilisée et de la durée de l'hydrolyse .

L'hydrolysat ainsi obtenu peut être utilisé pour la production de protéines d'intérêt (cellulases) par des microorganismes dont les gènes codant sont sous le contrôle d'un promoteur inductible par le sophorose. En particulier, le microorganisme est du genre *Trichoderma* et de préférence *Trichoderma reesei.*

On peut utiliser tout type de procédé de production adapté au microorganisme connu de l'homme du métier. En particulier, l'hydrolysat contenant le sophorose est mis en oeuvre dans un procédé de type "alimentation sans soutirage" ou "fed-batch" selon la terminologie anglo-saxonne comme décrit dans le document FR 2 555 603.

A cette fin, on prépare une préculture contenant le microorganisme et un milieu de culture renfermant des sels minéraux et des compléments vitaminiques usuels et une source de carbone et d'énergie de préférence sous forme de sucres solubles (par exemple le lactose). La préculture est ensuite transférée dans un fermenteur de production d'enzymes qui comprend un milieu de culture contenant au moins un sucre et supplémenté de manière régulière avec une solution inductrice. Dans le cadre de l'invention, la solution inductrice utilisée est l'hydrolysat issu de l'hydrolyse de sophorolipides. On procède ensuite à la production des enzymes dans le fermenteur en maintenant le contact nécessaire entre le microorganisme, le milieu de culture et en apportant régulièrement (par exemple en continu) la solution inductrice de sophorose.

Avant utilisation, il est également possible de purifier le mélange (hydrolysat) issu de l'étape d'hydrolyse afin d'isoler le sophorose produit. A cette fin, on ajoute dans ledit mélange un microorganisme capable de fermenter le glucose en éthanol, tel que par exemple la levure *Saccharomyces cerevisiae* et l'on procède ensuite à une étape de fermentation. Le mélange final obtenu après fermentation est soumis à une étape de stripage de l'éthanol afin de récupérer une solution aqueuse de sophorose. Il est également possible d'utiliser la solution aqueuse de sophorose ainsi obtenue comme solution inductrice d'expression de gènes chez des microorganismes.

### Description des figures

La figure 1 représente la concentration en cellulases produites en présence de glucose, de lactose et de sophorolipide, seuls ou en mélange.
La figure 2 représente l'évolution des concentrations en glucose et sophorose en fonction de la concentration en acide sulfurique mis en oeuvre pour l'hydrolyse ménagée de sophorolipides.
La figure 3 compare les vitesses spécifiques de production de cellulase par *Trichoderma reesei,* lors d'une production avec alimentation en fed-batch par une solution comprenant du glucose et du lactose ou sophorose.

### Exemples

### Exemple 1 (non-conforme)

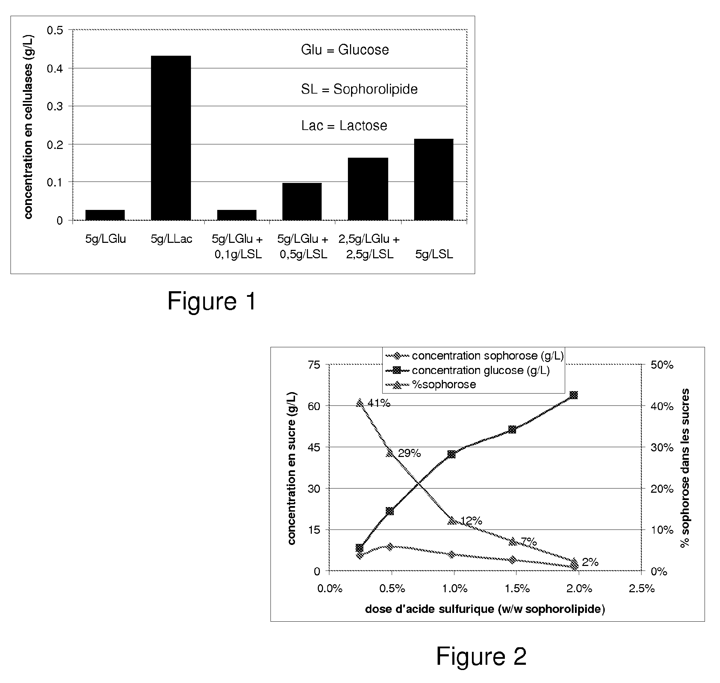

Après croissance de *T. reesei* en fiole sur 10 g/L de glucose et jusqu'à épuisement du glucose, on obtient un milieu contenant 5 g/L de biomasse (cellules de *T. reesei).* Dans ce milieu on réalise des injections de solutions concentrées de glucose et de sophorolipide sous forme acide issu d'une culture de *Candida bombicola* afin d'obtenir les teneurs désirées en glucose et sophorolipide indiquées dans la figure 1. On a également procédé à des injections de glucose seul, de lactose seul (inducteur industriel classique) et de sophorolipide seul qui correspondent à des milieux témoin.

Après 20h d'incubation, on analyse la quantité de protéines (cellulases) produites dans les différents milieux testés. Le dosage des protéines est effectué selon la méthode de Lowry OH et al. (1951) [Protein measurement with the Folin phénol reagent. Journal of Biological Chemistry 193, 1, 265-275] au moyen du kit DC Protein Assay (Biorad).

En référence à la figure 1, les résultats montrent que la quantité d'enzymes produites en présence de sophorolipides est toujours plus faible qu'en présence de lactose et donc que le sophorolipide brut est un moins bon inducteur que le lactose.

### Exemple 2 (optimisation des conditions de l'hydrolyse acide ménagée du sophorolipide)

Dans des tubes à essai, on mélange 1mL d'une solution aqueuse de sophorolipide (issu d'une culture de *Candida bombicola)* sous forme acide à environ 250 g/L et de l'acide sulfurique concentré, de manière à obtenir une concentration d'acide sulfurique comprise entre 0,25 et 2% poids d'acide par rapport au poids de sophorolipide.

Les tubes à essai sont bouchés avec un bouchon hermétique puis placés dans un bain sec à 100°C pendant 16 heures. Les concentrations en sucres (glucose et sophorose) libérés lors de l'hydrolyse sont mesurés par HPLC.

En référence à la figure 2, on observe que lorsque l'on augmente la teneur en acide sulfurique la libération de sucres totaux est améliorée (pour une même durée d'hydrolyse), mais cependant le mélange contient majoritairement du glucose. Au contraire pour de plus faibles teneurs en acide sulfurique, i.e. entre 0,1 et 1% poids d'acide sulfurique par rapport au poids de sophorolipide, le mélange après hydrolyse contient moins de sucres totaux mais une proportion de sophorose beaucoup plus importante (41% poids de sophorose dans le mélange hydrolysé avec 0,25% poids d'acide sulfurique par rapport au poids de sophorolipides).

Une expérience identique réalisée pendant 5 heures avec 8% poids d'acide sulfurique par rapport au poids de sophorolipide a entrainé une libération de glucose uniquement sans libération de sophorose associée.

### Exemple 3 (hydrolyse ménagée de sophorolipide)

On prépare 200 mL d'une solution contenant environ 200 g/L de sophorolipides sous forme acide et 0,25% d'acide sulfurique rapporté au poids de sophorolipide. Cette solution est mise à bouillir dans un ballon sous agitation et reflux pendant 12 heures. La fraction huile contenant les lipides résultant de l'hydrolyse est séparée de la phase aqueuse et les sucres (glucose et sophorose) libérés dans la fraction aqueuse sont dosés par HPLC. Les concentrations mesurées sont de 10,3 g/L de sophorose et de 16,6 g/L de glucose, soit 38% poids de sophorose dans le mélange de sucres libérés.

### Exemple 4 (utilisation de l'hydrolysat de sophorolipide pour l'induction de la production de cellulases par T. reesei)

Après une croissance de cellules de T. reesei CL847 *(*Durand H. et al. (1988a) Classical and molecular genetics applied to Trichoderma reesei for the selection of improved cellulolytic industrial strains. In: FEMS Symposium n°43: Biochemistry and Genetics of Cellulose Degradation, p. 135-151. Academic Press, Lond*on)* sur glucose en fioles agitées jusqu'à épuisement du glucose, la production de cellulases est réalisée selon un procédé "fed-batch" mettant en oeuvre une solution dont la composition est la suivante: 50 g/L d'un mélange de sucres et 15 mL/L NH₃ à 20% poids comme décrite dans la publication "A new stoichiometric miniaturization strategy for screening of industrial microbial strains: application to cellulase hyper-producing Trichoderma reesei strains", Microbial Cell Factories 2012, 11:70*.*

Le mélange de sucres utilisé contient (100-x)% poids de glucose et x% poids de sucre inducteur:
- dans le 1^{er} cas (témoin), le sucre inducteur est du lactose, à une teneur x=8%
- dans les 2^{ème}, 3^{ème} et 4^{ème} cas, le sucre inducteur est du sophorose provenant de l'hydrolysat obtenu à l'exemple 3 qui a été supplémenté en glucose pur de manière à avoir respectivement x=8%, x=0,3% et x=0,03%.

Le fed-batch est réalisé pendant 166 heures avec un débit d'alimentation en solution de 0,3 mL/h et à une température 30°C.

Des prélèvements journaliers sont effectués pour suivre la concentration en cellules et la concentration en protéines. La capacité d'induction du mélange de sucres utilisé pour le fed-batch est exprimée par la vitesse spécifique de production de protéines (mg de protéines produites par gramme de cellules productrices et par heure).

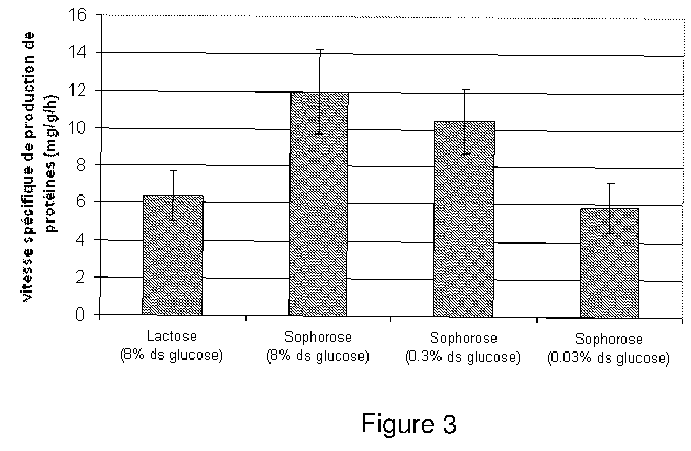

On observe sur la figure 3 que le mélange contenant du sophorose provoque une induction environ deux fois plus forte que le mélange contenant du lactose à même concentration, avec respectivement des vitesses spécifiques de production de protéines de 12 contre 6,4 mg/g/h.

De plus, on remarque sur la figure 3 que le mélange contenant 0,03% de sophorose provoque déjà une induction équivalente au mélange contenant 8% de lactose, avec des vitesses spécifiques de production de protéines de l'ordre de 6 mg/g/h.

Enfin, on observe sur la figure 3 que le mélange contenant 0,3% de sophorose provoque une induction équivalente au mélange contenant 8% de sophorose, avec respectivement des vitesses spécifiques de production de protéines de 10,5 et 12 mg/g/h.

## Revendications

1. Procédé de production de sophorose à partir de sophorolipides dans lequel :
a) on fournit une solution aqueuse de sophorolipides;
b) on ajoute un composé acide dans la solution aqueuse de sophorolipides, la teneur en composé acide étant comprise entre 0,01% et 6% poids par rapport au poids de sophorolipides
c) on chauffe le mélange obtenu à l'étape b) pendant 2 à 200 heures et à une température comprise entre 60 et 110°C.

2. Procédé selon la revendication 1, dans lequel la teneur en sophorolipides de la solution aqueuse est comprise entre 2 et 800 g/L.

3. Procédé selon la revendication 1 ou 2, dans lequel la teneur en composé acide est comprise entre 0,1 et 2% poids par rapport au poids de sophorolipides.

4. Procédé selon l'une des revendications précédentes, dans lequel le composé acide est un acide inorganique ou un acide organique.

5. Procédé selon la revendication 4 dans lequel l'acide inorganique est choisi parmi l'acide sulfurique, l'acide chlorhydrique, l'acide phosphorique, l'acide nitrique.

6. Procédé selon l'une des revendications précédentes, dans lequel on effectue une séparation de la phase lipide et de la phase aqueuse du mélange issu de l'étape c) de manière à récupérer un hydrolysat aqueux contenant du sophorose et du glucose.

7. Procédé de purification du sophorose d'un hydrolysat obtenu selon le procédé de la revendication 6 comprenant les étapes suivantes:
a) on ajoute un microorganisme capable de fermenter le glucose en éthanol dans l'hydrolysat ;
b) on effectue une fermentation éthanolique de l'hydrolysat de manière à fournir un hydrolysat fermenté;
c) on effectue un stripage de l'éthanol présent dans l'hydrolysat fermenté.

## Patentansprüche

1. Verfahren zum Herstellen von Sophorose ausgehend von Sophorolipiden, wobei:
a) man eine wässrige Sophorolipidlösung bereitstellt,
b) man eine saure Verbindung zu der wässrigen Sophorolipidlösung hinzufügt, wobei der Gehalt an saurer Verbindung zwischen 0,01 und 6 Gew.-% in Bezug auf das Gewicht an Sophorolipiden liegt,
c) man das bei Schritt b) erhaltene Gemisch während 2 bis 200 Stunden bei einer Temperatur zwischen 60 und 110 °C mischt.

2. Verfahren nach Anspruch 1, wobei der Gehalt an Sophorolipiden der wässrigen Lösung zwischen 2 und 800 g/l liegt.

3. Verfahren nach Anspruch 1 oder 2, wobei der Gehalt an saurer Verbindung zwischen 0,1 und 2 Gew.-% in Bezug zu dem Gewicht an Sophorolipiden liegt.

4. Verfahren nach einem der vorhergehenden Ansprüche, wobei die saure Verbindung eine anorganische Säure oder eine organische Säure ist.

5. Verfahren nach Anspruch 4, wobei die anorganische Säure aus Schwefelsäure, Salzsäure, Phosphorsäure, Salpetersäure ausgewählt wird.

6. Verfahren nach einem der vorhergehenden Ansprüche, wobei man eine Abscheidung der Lipidphase und der wässrigen Phase des Gemischs, das aus Schritt c) hervorgeht, derart ausführt, dass wässriges Hydrolysat gewonnen wird, das Sophorose und Glukose enthält.

7. Verfahren zum Reinigen der Sophorose eines Hydrolysats, das gemäß dem Verfahren des Anspruchs 6 erhalten wird, das die folgenden Schritte aufweist:
a) man fügt einen Mikroorganismus hinzu, der fähig ist, die Glukose in dem Hydrolysat in Ethanol zu fermentieren,
b) man führt eine Ethanolfermentation des Hydrolysats derart aus, dass ein fermentiertes Hydrolysat geliefert wird,
c) man führt ein Strippen des Ethanols, das in dem fermentierten Hydrolysat vorhanden ist, aus.

## Claims

1. A process for the production of sophorose starting from sophorolipids, in which:
a) an aqueous solution of sophorolipids is provided;
b) an acidic compound is added to the aqueous solution of sophorolipids, the quantity of the acidic compound being in the range 0.01% to 6% by weight with respect to the weight of sophorolipids;
c) the mixture obtained in step b) is heated for 2 to 200 hours and at a temperature in the range 60°C to 110°C.

2. The process according to claim 1, in which the quantity of sophorolipids in the aqueous solution is in the range 2 to 800 g/L.

3. The process according to claim 1 or claim 2, in which the quantity of acidic compound is in the range 0.1% to 2% by weight with respect to the weight of sophorolipids.

4. The process according to one of the preceding claims, in which the acidic compound is an inorganic acid or an organic acid.

5. The process according to claim 4, in which the inorganic acid is selected from sulphuric acid, hydrochloric acid, phosphoric acid and nitric acid.

6. The process according to one of the preceding claims, in which a step for separation of the lipid phase and the aqueous phase of the mixture obtained from step c) is carried out in order to recover an aqueous hydrolysate containing sophorose and glucose.

7. A process for the purification of sophorose from a hydrolysate obtained according to the process of claim 6, comprising the following steps:
a) adding a microorganism which is capable of fermenting the glucose into ethanol in the hydrolysate;
b) carrying out an ethanolic fermentation of the hydrolysate in order to provide a fermented hydrolysate;
c) stripping the ethanol present in the fermented hydrolysate.
